(19) **Europäisches Patentamt European Patent Office Office européen des brevets**

(11) **EP 4 613 197 A1**

(12) **EUROPEAN PATENT APPLICATION**

(43) Date of publication:
**10.09.2025 Bulletin 2025/37**

(21) Application number: **25158711.9**

(22) Date of filing: **19.02.2025**

(51) International Patent Classification (IPC):
*A61B 5/16* (2006.01)   *A61B 5/372* (2021.01)
*A61B 5/397* (2021.01)   *A61B 5/398* (2021.01)
*A61B 5/00* (2006.01)   *G06N 20/00* (2019.01)

(52) Cooperative Patent Classification (CPC):
**A61B 5/16; A61B 5/168; A61B 5/372; A61B 5/397;
A61B 5/398; A61B 5/7267; G06N 20/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.03.2024   CN 202410253772**

(71) Applicant: **Robert Bosch GmbH
70442 Stuttgart (DE)**

(72) Inventors:
• **XIA, Lijuan
  Shanghai, 200335 (CN)**
• **ZHANG, Shouyi
  Shanghai, 200335 (CN)**
• **YU, Yifei
  Shanghai, 200335 (CN)**
• **SUN, Zhigang
  Shanghai, 200335 (CN)**

(74) Representative: **Isarpatent
Patent- und Rechtsanwälte Partg mbB
Friedrichstraße 31
80801 München (DE)**

(54)   **METHODS AND DEVICES FOR DETECTING THE ALERTNESS OF THE HUMAN BODY**

(57)   The present invention provides a method for detecting the alertness of the human body. The method comprises receiving a bioelectric signal for detection of the alertness of the human body; using an alertness feature extraction model trained by a self-supervised learning method to obtain the feature data of the bioelectric signal for detection of the alertness of the human body; utilizing the alertness detection model for detection of the alertness of the human body based on the feature data of the bioelectric signal.

FIG. 4

EP 4 613 197 A1

**Description**

**Technical Field**

The present invention is generally related to techniques for detecting the alertness of the human body, and more particularly to techniques for detecting alertness based on bioelectric signals of the human body using deep machine learning models.

**Background Art**

In people's daily lives and production operations, physical fatigue or inattention can lead to significant safety hazards, resulting in loss of life and property. This is due to the inattention, slow reactions, and inaccurate judgments that occur when the human body is operating under fatigue, resulting in accidents due to operational errors. Thus, monitoring the level of fatigue of the human body in scenarios such as driving, working with live wires, working at heights, etc. is important for safe living and production.

Currently, it is a common practice to detect whether the human body is in a state of fatigue based on the brain electrical signals (e.g., EEG) of the human body. This detection method based on the physiological signals of the human body has advantages including strong objectivity and high accuracy. With the development of artificial intelligence technology and the improvement of computer processing capabilities, there have also been many approaches in recent years that have utilized traditional or deep machine learning models to judge whether the human body is in a state of fatigue based on the EEG signals of the human body. However, the performance of current machine learning models for fatigue detection across subjects relies heavily on the quality of the feature data extracted from the EEG signal. That is, a machine learning model trained based on a data set that includes fatigue sample data from a plurality of human bodies may not provide good performance for the detection of fatigue of human bodies outside the data set. Accordingly, there is a need for a method that is capable of improving the performance of fatigue detection across subjects.

**Summary of Invention**

A brief description of one or more aspects according to the present disclosure is provided below in order to provide a basic understanding of these aspects. The present disclosure is not a broad overview of all aspects, nor is it intended to identify the key elements of all aspects or delineate the scope of any or all aspects. Its sole purpose is to present some concepts of one or more aspects in a simplified form as a preamble to the more detailed description that follows.

According to one aspect of the present disclosure, a method is provided for detecting the alertness of the human body. The method comprises: obtaining a data set comprising a plurality of samples, each of which comprises a bioelectric signal of the human body and a corresponding alertness value; generating a positive sample pair based on the data set, the positive sample pair comprising a first sample randomly sampled from the data set and a second sample with the same alertness level as the first sample; training, by way of self-supervised learning, an alertness feature extraction model with the positive sample pair such that the alertness feature extraction model minimizes the distance in the alertness feature space between the first and second features extracted from the first and second samples, respectively.

According to one aspect of the present disclosure, a method is provided for detecting the alertness of the human body. The method comprises: receiving a bioelectric signal for detection of the alertness of the human body; using an alertness feature extraction model trained by a self-supervised learning method to extract from the bioelectric signal the feature data for detection of the alertness of the human body; utilizing the alertness detection model for detection of the alertness of the human body based on the feature data of the bioelectric signal.

According to one aspect of the present disclosure, a device is provided for detecting the alertness of the human body. The device comprises: a receiving module for receiving a bioelectric signal for detection of the alertness of the human body; a feature extraction module for using an alertness feature extraction model trained by a self-supervised learning method to extract from the bioelectric signal the feature data for detection of the alertness of the human body; a detection module for utilizing the alertness detection model for detection of the alertness of the human body based on the feature data of the bioelectric signal.

According to another aspect of the present disclosure, a device is provided for detecting the alertness of the human body. The device may comprise a memory and at least one processor coupled to the memory. The at least one processor may be configured to: obtain a data set comprising a plurality of samples, each of which comprises a bioelectric signal of the human body and a corresponding alertness value; generate a positive sample pair based on the data set, the positive sample pair comprising a first sample randomly sampled from the data set and a second sample with the same alertness level as the first sample; train, by way of self-supervised learning, an alertness feature extraction model with the positive sample pair such that the alertness feature extraction model minimizes the distance in the alertness feature space between the first and second features extracted from the first and second samples, respectively.

According to another aspect of the present disclosure, a device is provided for detecting the alertness of the human body. The device may comprise a memory and at least one processor coupled to the memory. The at least one processor may be configured to: receive a bioelectric signal for detection of the alertness of the human body; use an alertness feature extraction model trained by a self-supervised learning method to extract from the bioelectric signal the feature data for detection of the alertness of the human body; utilize the alertness detection model for detection of the alertness of the human body based on the feature data of the bioelectric signal.

According to another aspect of the present disclosure, a computer-readable medium is provided that stores computer program code for detecting the alertness of the human body. The computer program code, when executed by the processor, may cause the processor to obtain a data set comprising a plurality of samples, each of which comprises a bioelectric signal of the human body and a corresponding alertness value; generate a positive sample pair based on the data set, the positive sample pair comprising a first sample randomly sampled from the data set and a second sample with the same alertness level as the first sample; train, by way of self-supervised learning, an alertness feature extraction model with the positive sample pair such that the alertness feature extraction model minimizes the distance in the alertness feature space between the first and second features extracted from the first and second samples, respectively.

According to another aspect of the present disclosure, a computer-readable medium is provided that stores computer program code for detecting the alertness of the human body. The computer program code, when executed by the processor, may cause the processor to receive a bioelectric signal for detection of the alertness of the human body; use an alertness feature extraction model trained by a self-supervised learning method to extract from the bioelectric signal the feature data for detection of the alertness of the human body; utilize the alertness detection model for detection of the alertness of the human body based on the feature data of the bioelectric signal.

According to another aspect of the present disclosure, a computer program product is provided for detecting the alertness of the human body. The computer program product may comprise processor-executable computer program code. The processor-executable computer program code is used for: obtaining a data set comprising a plurality of samples, each of which comprises a bioelectric signal of the human body and a corresponding alertness value; generating a positive sample pair based on the data set, the positive sample pair comprising a first sample randomly sampled from the data set and a second sample with the same alertness level as the first sample; training, by way of self-supervised learning, an alertness feature extraction model with the positive sample pair such that the alertness feature extraction model minimizes the distance in the alertness feature space between the first and second features extracted from the first and second samples, respectively.

According to another aspect of the present disclosure, a computer program product is provided for detecting the alertness of the human body. The computer program product may comprise processor-executable computer program code. The processor-executable computer program code is used for: receiving a bioelectric signal for detection of the alertness of the human body; using an alertness feature extraction model trained by a self-supervised learning method to extract from the bioelectric signal the feature data for detection of the alertness of the human body; utilizing the alertness detection model for detection of the alertness of the human body based on the feature data of the bioelectric signal.

It should be noted that the above one or more aspects include the following detailed description and features that are specifically recorded in the Claims. The following specification and accompanying drawings detail some of the exemplary features from a variety of aspects. These features merely indicate a variety of ways in which the principles of various aspects may be implemented, and the present disclosure is intended to include all of these aspects and their equivalents.

**Description of Accompanying Drawings**

The following drawings describe various examples of the present disclosure for illustrative purposes only. Those skilled in the art will readily recognize from the following description that alternative examples of the methods and structures disclosed herein may be achieved without departing from the spirit and principles disclosed herein.

FIG. 1 illustrates a schematic diagram of an EEG signal for detecting the alertness of the human body according to one example of the present disclosure.

FIG. 2 illustrates a flowchart of a method for detecting the alertness of the human body according to one example of the present disclosure.

FIG. 3 illustrates a schematic diagram of random transform processing for detecting the alertness of the human body according to one example of the present disclosure.

FIG. 4 illustrates a schematic diagram of a method for self-supervised learning of extracting human body alertness features according to one example of the present disclosure.

FIG. 5 illustrates a schematic diagram of an alertness feature extraction model according to one example of the present disclosure.

FIG. 6 illustrates a flowchart of a method for detecting the alertness of the human body according to one example of the present disclosure.

FIG. 7 illustrates a schematic diagram of an alertness detection model according to one example of the present disclosure.

FIG. 8 illustrates a block diagram of a device for detecting the alertness of the human body according to one example of the present disclosure.

FIG. 9 illustrates a block diagram of a device for detecting the alertness of the human body according to one example of the present disclosure.

## Specific Embodiments

In the following description, numerous specific details are set forth to provide a thorough understanding of the examples of the present invention. However, those skilled in the relevant art will recognize that the invention can be practiced without one or more of the specific details, or by using alternative methods, components, etc., to practice the invention. In some instances, well-known structures and operations are not shown or described in detail to avoid unnecessarily obscuring the present invention.

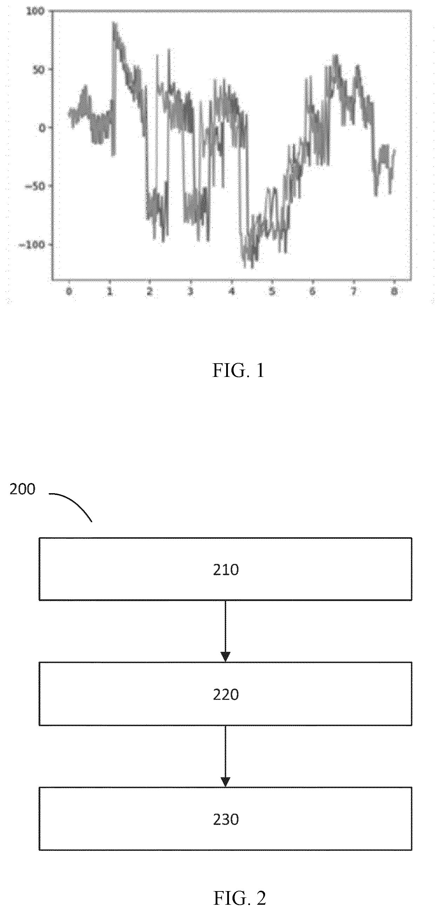

FIG. 1 illustrates a schematic diagram of an EEG signal for detecting the alertness of the human body according to one example of the present disclosure. Electroencephalography (EEG) signals generally refer to human scalp EEG, which is a map of the relationship between scalp potential difference and time. EEG signals directly reflect the electrical activity of brain tissue and can be used to assess the alertness of the human body. Alertness is associated with, among other things, the degree of fatigue and the degree of mental concentration of the human body, i.e., the degree of alertness may also reflect the degree of fatigue and/or mental concentration etc. of the human body. The devices used to record EEGs have developed from the original 1 or 2 channels to the now commonly used 16, 32, or 64 channels, and can even collect EEG signals from 512 scalp electrodes. The greater the number of channels, the greater the amount of information collected, and the more accurate the alertness detection result based on that information. The present disclosure is illustrated by a single-channel EEG signal collected by a forehead electrode as an example as high alertness detection performance can be achieved by the present disclosure scheme even on the basis of a single-channel EEG signal alone, but the present disclosure is not limited to a specific method of EEG signal collection.

The vertical axis shown in FIG. 1 is the potential difference of the EEG signal in microvolts ($\mu$V). As can be seen from FIG. 1, the potential difference in the EEG signal is very low, typically less than 100'$\mu$V, and it is therefore susceptible to interference from surrounding electromagnetic field radiation and the electronic noise within the instrument during the acquisition process. The horizontal axis of FIG. 1 is the time axis of the EEG signal in seconds (s). FIG. 1 illustrates the EEG signal graph within an 8 s time window. The time window may be a basic unit of time of an EEG signal for detecting the alertness of the human body. In other examples, the length of the time window may also be other values between 4 s and 12 s and may even be a length of time less than 4 s or greater than 12 s. The length of the time window may be determined based on the specific application scenario and design needs. For example, in scenarios where the results of the test need to be output quickly and frequently, the time window may be set to a shorter length; in scenarios where more accurate and reliable test results are needed, the time window may be set to a longer length.

In addition to EEG signals, bioelectric signals that reflect the physiological state of the human body, such as electrooculography signals (EOG) and electromyography signals (EMG), can also be used to detect the alertness of the human body. For example, an EOG signal comprises signals that reflect the eye movement characteristics of the human body, such as eye movement, blinking frequency, etc., which can reflect the alertness of the human body. EMG signals can reflect the movement intent and muscle fatigue of the human body and are therefore also capable of being used to detect the alertness of the human body. In various examples of the present disclosure, on various combinations of bioelectric signals of the human body, such as EEG signals, EOG signals, and EMG signals, may be used for detecting the alertness of the human body.

FIG. 2 illustrates a flowchart of a method 200 for detecting the alertness of the human body according to one example of the present disclosure. The method 200 may be a method for training an alertness feature extraction model for extracting feature data for alertness detection based on a bioelectric signal of the human body. In block 210, the method 200 may first obtain a data set comprising a plurality of samples. Each of the plurality of samples may comprise a bioelectric signal of the human body and a corresponding alertness value.

The alertness value may correspond to a bioelectric signal of the human body in units of time windows as shown in FIG. 1. This alertness value may be a specific value that represents the degree of alertness (e.g., degree of fatigue and/or degree of concentration) of the human body, which may be a natural number, an integer, a rational number, a real number, etc. The type and range of values of the alertness value taken may depend on the data set. The data set may be an existing open data set. The data set can also be obtained by: using a bioelectric signal collection device to collect a bioelectric signal of the detected human body and the alertness value of the detected human body is either manually labeled or automatically generated using computer vision processing techniques for facial expressions etc. In one example, the alertness value may be any rational number between 0 and 1.

In another example, the alertness value may be any natural number between 1 and 100. The present disclosure will be described using a data set of alertness values comprising real numbers ranging from 0 to 1 as an example, but the present disclosure is not limited to a specific type and range of alertness values.

In block 220, the method 200 may generate a positive sample pair based on the obtained data set. The positive sample pair may comprise a first sample randomly sampled from the data set and a second sample with the same alertness level as the first sample. That the first sample and the second sample have the same alertness level means that the alertness values of the first sample and the second sample are the same or are in the same value range.

In one example, generating a positive sample pair based on the obtained data set may comprise: dividing the samples in the data set into a plurality of sample groups with different alertness levels based on the alertness values of the samples in the data set. Where the number of samples with different alertness values in the data set is very unevenly distributed, e.g., when collecting the data set, there are usually more samples with the detected human body being in a high alertness state and fewer samples in a low alertness state, samples in the data set may be sorted by alertness value and then grouped in fixed numbers of samples. This can allow each sample group to comprise the same number of samples, thereby avoiding potential problems that include sample groups with lower numbers of samples. Where the number of samples with different alertness values in the data set is more evenly distributed, the alertness values can also be divided into multiple continuous numerical intervals of the same length, and then samples in the data set are grouped based on the assigned numerical intervals, i.e., samples with alertness values within the same numerical interval are divided into the same sample group. Regardless of the manner in which the samples are grouped, samples in the same sample group should have the same or similar alertness values, i.e., they may be referred to as having the same alertness level.

The step of generating a positive sample pair after grouping samples in the data set may comprise randomly sampling a first sample from a plurality of sample groups and randomly sampling a second sample from the sample group where the first sample is located. The first and second samples have the same alertness level and may be referred to as a positive sample pair.

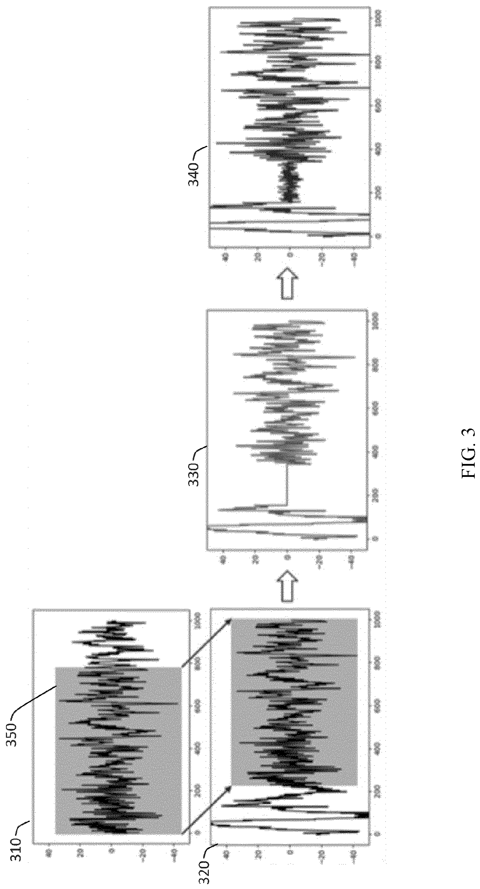

In another example, a positive sample pair may be generated by random transform processing as described below in conjunction with FIG. 3. For example, generating a positive sample pair based on the obtained data set may comprise: A first sample in a positive sample pair is obtained by randomly sampling from a data set. The bioelectric signal of the second sample in the positive sample pair is obtained by random transform processing of the bioelectric signal in the first sample, for example, by one or more of shifting, trimming, scaling, or adding noise, and the second sample can maintain the same alertness value as the alertness value in the first sample (that is, the above random transform processing does not change the essential characteristics of the bioelectric signal and thus does not change the alertness value of the human body reflected by the bioelectric signal).

In block 230, the method 200 can train, by way of self-supervised learning, an alertness feature extraction model such that the distance between the features extracted by the alertness feature extraction model for the samples in the positive sample pair, such as the first feature and the second feature extracted from the first sample and the second sample, in the alertness feature space is minimized. The method 200 may utilize a plurality of positive sample pairs generated in block 220 to optimize the parameters in the alertness feature extraction model through a stochastic gradient descent algorithm to minimize the distance in the alertness feature space between the features extracted from the positive sample pair.

Self-supervised learning (SSL) is a machine learning paradigm for processing unlabeled data to obtain useful data features. Since the above examples of self-supervised learning using positive sample pairs composed of first samples randomly sampled from the data set and second samples randomly transformed from the data set are designed to minimize the distance in the alertness feature space between the features extracted from the first sample and the second sample and not utilize the specific alertness values of the samples, the features learned and extracted in this manner have smaller deviations between different subjects or data sets, are better able to reflect the essential features of alertness, and are less affected by the differences in the relationship between the bioelectric signals and alertness values of different subjects. Thus, they can be used to provide more accurate alertness detection for subjects outside the data set. In the implementation of self-supervised learning using the above positive sample pairs of sample-based alertness values, the alertness value labels are likewise not utilized during the optimization of the alertness feature extraction model. Instead, the alertness values are only used as a guide in the process of generating positive sample pairs. Therefore, this method of self-supervised learning may also be referred to as label-guided self-supervised learning in the present disclosure.

Because the machine learning methods of the present disclosure are capable of extracting more useful alertness feature data from bioelectric signals of the human body, the alertness feature extraction model can adopt a simpler model architecture. In one example, the alertness feature extraction model may be a one-dimensional convolutional neural network (1-D CNN). This lightweight alertness feature extraction model can be implemented on a less expensive processor or chip with lower power consumption, enabling a significant reduction in product costs without substantially reducing performance.

In block 220, the method 200 may further generate a negative sample pair based on the data set. In one example, the negative sample pair may include a third sample and a fourth sample randomly sampled from the data set. That is, for a sample randomly sampled from a data set, any other sample in that data set can constitute a negative sample pair with that sample. Since the two samples in the negative sample pair are randomly sampled from the data set, there are differences associated with the distribution of samples in the data set. In another example, the two samples in the negative sample pair may also be further limited to having different alertness values or different alertness levels (e.g., having alertness values at different value intervals). Accordingly, in block 230, the alertness feature extraction model can be trained by self-supervised learning using positive and negative sample pairs such that the distance between the first feature and the second feature extracted by the alertness feature extraction model from the first sample and the second sample in the positive sample pair in the alertness feature space is minimized and the distance between the third feature and the fourth feature extracted by the alertness feature extraction model from the third sample and the fourth sample in the negative sample pair in the alertness feature space is maximized. By further using a negative sample pair to train the alertness feature extraction model, it can help the alertness feature extraction model learn alertness features with better performance.

Although not shown in FIG. 2, in some examples, prior to block 230, the method 200 may further comprise random transform processing of samples in one or more positive sample pairs and negative sample pairs generated in block 220 for training the alertness feature extraction model. Random transform processing may include one or more of shifting, trimming, scaling, and adding noise.

FIG. 3 illustrates a schematic diagram of random transform processing for detecting the alertness of the human body according to one example of the present disclosure. The ordinate in the EEG signal graphs 310-340 in FIG. 3 represents the potential difference of the EEG signal and its unit is microvolt ($\mu$V). Unlike FIG. 1, the abscissa in the EEG signal graphs 310-340 in Figure 3 represents the sampling point index. In this example, the time window length of the EEG signal for each sample in the data set is 8 s. Since the frequency of a valid EEG signal is typically in the range of less than 50 Hz, a sampling frequency of more than twice 50 Hz can be employed. In this example, a sampling frequency of 125 Hz is employed, so the EEG signal within the 8 s time window can have 1000 sampling points as shown in FIG. 3.

The EEG signal graph 310 illustrates the EEG signal in the raw sample. The EEG signal graph 320 illustrates the resulting EEG signal after the raw EEG signal is shifted. The shifting operation may include sliding the time window to the left (i.e., the time direction preceding) or to the right (i.e., the time direction following) a random length of time based on the original complete time sequence EEG signal to obtain the shifted EEG signal. The length of time to shift should generally be less than one quarter of the time window length. For example, the shift length of the sample signal for 1000 sampling points should be fewer than 250 sampling points. In the example of FIG. 3, by sliding the time window to the left slightly more than 200 sampling point time units (i.e., slightly more than 1.6 s) so that the portion of the raw EEG signal covered by the gray box 350 remains in the time window, a later portion of the raw EEG signal is moved out of the time window and an earlier portion of the raw EEG signal is moved into the time window, resulting in a shifted EEG signal as shown in EEG signal graph 320.

EEG signal graph 330 illustrates the EEG signal obtained after trimming the EEG signal in the EEG signal graph 320 (i.e., the shifted EEG signal). The trimming processing may comprise setting signals of random length in the EEG signal to a predetermined range of values at one or more locations (typically no more than 4 locations). The sum of the random lengths of the plurality of locations should generally be less than a quarter of the time window length. For example, the total number of sampling points trimmed should be fewer than 250 for a sample signal of 1000 sampling points. As shown in the EEG signal graph 330, the voltage value of the sampling points with indices of approximately 100 to 300 in the EEG signal is trimmed to 0.

EEG signal graph 340 illustrates the EEG signal obtained after scaling and adding noises to the EEG signal in the EEG signal graph 330 (i.e., the shifted and trimmed EEG signal). The scaling processing may comprise proportionally amplifying or reducing the voltage values of all sampling points in the EEG signal. Adding noise may comprise random jittering of the voltage values of at least some of the sampling points in the EEG signal, such as superimposing a Gaussian noise signal. The amplitude (i.e., voltage value) of the EEG signal as shown in EEG signal graph 340 is amplified overall as compared to the amplitude of the EEG signal in EEG signal graph 330 and Gaussian noise is superimposed (as shown by the sampling points with indices of approximately 100 to 300).

In the random transform processing shown in FIG. 3, shifting, trimming, scaling, and adding noise are performed on the raw EEG signal sample. In still further examples, the random transform processing may comprise only one or some of the operations of shifting, trimming, scaling, and adding noise and processing may be in a different order than that shown in FIG. 3. Further, random transform processing may also comprise other processing operations that make minor changes to the EEG signal but do not alter the intrinsic features of the EEG signal.

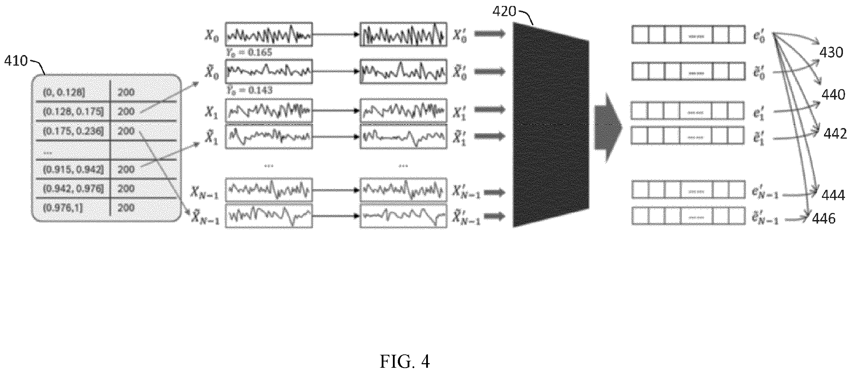

FIG. 4 illustrates a schematic diagram of a method for self-supervised learning of extracting human body alertness features according to one example of the present disclosure. This method of self-supervised learning may also be referred to as a label-guided self-supervised learning method. This method of self-supervised learning can be used to train an alertness feature extraction model to enable the trained alertness feature extraction model to extract higher quality features associated with alertness from the bioelectric signals of the human body for subsequent alertness detection and other related tasks.

Table 410 in FIG. 4 schematically illustrates a data set for training an alertness feature extraction model. Samples in this data set may be expressed as $\{X_i, Y_i\}$, $i = 0, ..., M - 1$, wherein $X_i$ represents the bioelectric signal of the human body for one sample in the data set, which may be a one-dimensional time sequence comprising 1000 sampling points, $Y_i$ represents an alertness value of the human body corresponding to that signal (i.e., the labeled true value, ground truth), which may be any real value between 0.0 and 1.0 (e.g., 0 for the awake state, 1 for the inactive state), and M represents the total number of samples in the data set.

The data set table 410 may also be referred to as a data set dictionary or sample dictionary, which is obtained by grouping samples based on alertness values (see the detailed description of block 220 in conjunction with FIG. 2), and each row in the table represents a sample group. In this example, the number of samples in each sample group can be set as a fixed value based on the total number of samples in the data set due to the very uneven distribution of samples with different alertness values in the data set, i.e., the number of samples with low alertness values is small. As shown in Table 410, each sample group includes the same number of samples, 200. The alertness value range corresponding to the sample group in the first row is (0, 0.128], the alertness value range corresponding to the sample group in the second row is (0.128, 0.175], the alertness value range corresponding to the sample group in the third row is (0.175, 0.236], ..., the alertness value range corresponding to the sample group in the fifth row is (0.915, 0.942], the alertness value range corresponding to the sample group in the sixth row is (0.942, 0.976], and the alertness value range corresponding to the sample group in the sixth row is (0.976, 1]. It can be seen that the lengths of the alertness value ranges to which the samples in each sample group belong are different. The higher the alertness value of the sample, the smaller the alertness value range of the sample group in which it is located. Nonetheless, the samples in each sample group may still be considered to have the same or similar alertness values, i.e., the same alertness level.

The process of training the alertness feature extraction model 420 (e.g., the 1-D CNN) by a self-supervised learning method may comprise, in general terms, the following steps.

Step 1: One or more samples $X_0, X_1... ...X_{N-1}$ are randomly sampled from data set table 410, wherein N is the number of samples randomly sampled. N may be 32 or 64 or greater depending on the processing capabilities of the hardware (e.g., a centralized processor, graphics card, etc.). An additional sample is then randomly sampled from the sample group in which each randomly sampled sample is located, thereby forming a positive sample pair. For example, the alertness value $Y_0$ of the first sample $X_0$ is equal to 0.165, which is located in the sample group in the second row in the data set table 410. Therefore, the second sample $\tilde{X}_0$ can be randomly sampled from the sample group in the second row and its alertness value $\tilde{Y}_0$ is equal to 0.143, which belongs to the alertness value range (0.128, 0.175]. The first sample $X_0$ and the second sample $\tilde{X}_0$ can be called a positive sample pair. Similarly, samples $\tilde{X}_1... ...\tilde{X}_{-1}$ can be obtained, where $X_1$ and $\tilde{X}_1$ can be called a positive sample pair, $X_{N-1}$ and $\tilde{X}_{N-1}$ can be called a positive sample pair, and so on.

In this example, the sample $X_0$ may each form a negative sample pair with any other sample except sample $\tilde{X}_0$ from the samples sampled above (including $X_1, \tilde{X}_1, ... ..., X_{N-1}, \tilde{X}_{N-1}$), i.e., even if $X_1$ and $X_0$ have the same alertness value (e.g., 0.165) or their alertness values belong to the same value range (e.g., (0.128, 0.175], i.e., all in the sample group in the second row), $X_0$ and $X_1$ can constitute a negative sample pair. In another example, sample $X_0$ can constitute a negative sample pair with samples obtained by sampling as above (including $X_1, \tilde{X}_1, ... ... , X_{N^-1}, \tilde{X}_{N-1}$) having an alertness value different from $X_0$ or belonging to an alertness value range different from $X_0$, i.e., if 1 and 0 have the same alertness value, then 0 and 1 cannot constitute a negative sample pair, or if the alertness values of $X_1$ and $X_0$ belong to the same value range, then $X_0$ and $X_1$ cannot constitute a negative sample pair.

Step 2: After sampling to obtain a batch of samples $(X_0, \tilde{X}_0, X_1, \tilde{X}_1,... ... , X_{N-1}, \tilde{X}_{N-1})$, each sample or at least some of the samples in the batch of samples can be subjected to the random transform processing described in conjunction with FIG. 3 to obtain processed samples $(X'_0, \tilde{X}'_0, X'_1, \tilde{X}'_1,... ... , X'_{N-1}, \tilde{X}'_{N-1})$. Step 2 may be optional in the label-guided self-supervised learning method as shown in FIG. 4. Since the label-guided self-monitored learning method does not directly utilize the specific alertness values labeled in the samples during training (see Step 3) and samples in the positive sample pairs may be from different subjects, the bias based on the data set can be mitigated and similar alertness features can be learned between different subjects without relying on the specific alertness values of each subject in the data set, which can help provide more accurate alertness detection for subjects outside the data set. Since the differences between the alertness features of different subjects may be considered a kind of bioelectric signal noise, by adding the random transform processing in Step 2, the processed samples can be used to simulate the bioelectric signals of different subjects, which can further reduce cross-subject bias, thereby providing good performance for subjects outside the data set and improving the generalization ability of the alertness feature extraction model.

Step 3: Samples sampled in Step 1 $(X_0, \tilde{X}_0, X_1, \tilde{X}_1,... ... , X_{N-1}, \tilde{X}_{N-1})$ or samples processed in Step 2 $(X'_0, \tilde{X}'_0, X'_1, \tilde{X}'_1, ... ... , X'_{N-1}, \tilde{X}'_{N-1})$ are input into alertness feature extraction model 420. The alertness feature extraction model 420 can extract alertness features for each input sample from the bioelectric signal $(e'_0, \tilde{e}'_0, e'_1, \tilde{e}'_1,... ... , e'_{N-1}, \tilde{e}'_{N-1})$. Using a stochastic gradient descent algorithm, the parameters of the alertness feature extraction model 420 can be optimized by making the distances between the features extracted for the positive sample pairs in the alertness feature space (e.g., the distance 430 between $e'_0$ and $\tilde{e}'_0$ etc.) closer and making the distances between the features extracted for the negative sample pairs in the alertness feature space (e.g., the distance 440 between $e'_0$ and $e'_1$, the distance 442 between $e'_0$ and $\tilde{e}'_1$, the distance 444 between $e'_0$ and $e'_{N-1}$, the distance 446 between $e'_0$ and $\tilde{e}'_{N-1}$ etc.) farther. The loss function for optimizing the alertness feature extraction model 420 may be expressed as:

$$\mathcal{L} = \sum_{i=0}^{N-1} - \log \frac{\exp(e_i' \cdot \tilde{e}_i'/\tau)}{\sum_{j=0, j \neq i}^{N-1} \exp(e_i' \cdot \tilde{e}_j'/\tau) + exp(e_i' \cdot e_j'/\tau)}$$

Wherein both $i$ and $j$ are indices of the output alertness features and $\tau$ is the temperature coefficient used to control the loss function constraint density. Steps 1-3 may be repeated until the loss function converges or the predetermined threshold is met.

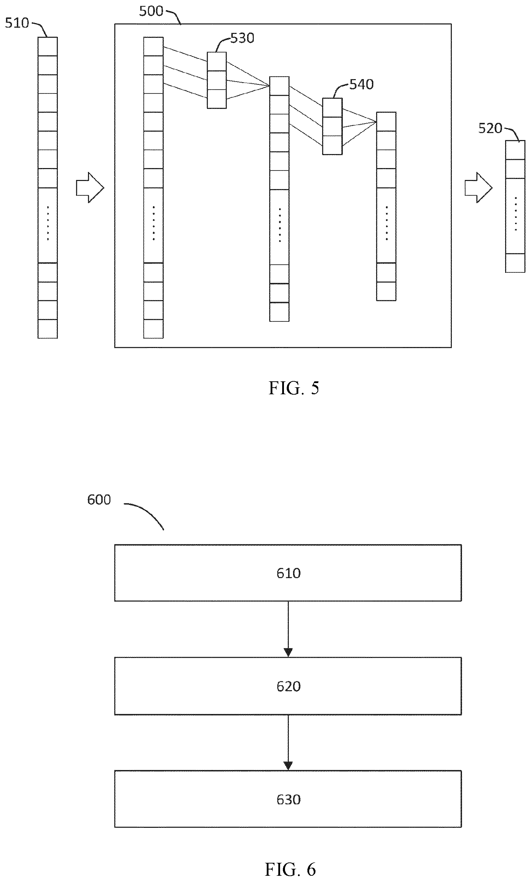

FIG. 5 illustrates a schematic diagram of an alertness feature extraction model according to one example of the present disclosure. In this example, the alertness feature extraction model may utilize a one-dimensional convolutional neural network (1-D CNN) 500. The input data 510 input into the one-dimensional convolutional neural network may be a bioelectric signal of the human body (e.g., an EEG signal) within a time window or a randomly transformed bioelectric signal of the human body (e.g., $X_0'$, $\tilde{X}_0'$, $X_1'$, $\tilde{X}_1'$, ......, $X_{N-1}'$, $\tilde{X}_{N-1}'$) The input data 510 may be a sequence of 1000 in length, represented by a vector. Depending on the time window length and the sampling frequency, the input data 510 may have different lengths.

The one-dimensional convolutional neural network 500 may comprise one or more convolutional layers. As shown in FIG. 5, in the first convolutional layer, convolution kernel 530 may be utilized to perform a convolution operation with the input data 510 and to input the results of the convolution to the next convolutional layer. In the next convolution layer, convolution kernel 540 may be utilized to perform a convolution operation with the convolution result output by the previous convolutional layer, and the convolution result is input to the next convolutional layer, and so on, until finally the output data 520 (i.e., the extracted features, e.g., $X_0'$, $\tilde{e}_0'$, $e_1'$, $\tilde{e}_1'$, ......, $e_{N-1}'$, $\tilde{e}_{N-1}'$) is obtained by the last layer. The number of convolutional layers can be used as a hyperparameter to be adjusted during training. The lengths of convolution kernels 530 and 540 and the sliding step lengths can be used as hyperparameters to be adjusted during the training. The values in the convolution kernels 530 and 540 are model parameters learned by the methods described above in conjunction with FIGS. 2-4. By utilizing a self-supervised learning method consistent with the present disclosure, the one-dimensional convolutional neural network 500 may have a lighter architecture with fewer parameters. Although not shown in FIG. 5, the one-dimensional convolutional neural network 500 may also comprise one or more other layers, such as pooling layers, fully connected layers, etc.

FIG. 6 illustrates a flowchart of a method 600 for detecting the alertness of the human body according to one example of the present disclosure. The method 600 may be performed by a device for detecting the alertness of the human body as described in the examples below or a wearable device having the functionality to detect human alertness. In block 610, the method 600 can receive a bioelectric signal for detecting the alertness of the human body. The bioelectric signal for detecting the alertness of the human body may comprise at least one of the EEG signal, EOG signal, EMG signal, etc. of the human body as described above. For example, the method 600 can detect the alertness of the human body based solely on the EEG signal of the human body. In particular, through the methods disclosed in the present disclosure, detecting the alertness of the human body based on single-channel EEG signals (for example, forehead EEG signals) can achieve or exceed the accuracy and reliability of existing methods for detecting the alertness of the human body. Of course, the method 600 can also detect the alertness of the human body based on a combination of a variety of bioelectric signals of the human body, such as EEG and EOG signals. The method 600 may acquire a corresponding bioelectric signal of the human body from existing bioelectric signal detection instruments or may utilize a bioelectric signal detection sensor integrated in a wearable device to acquire a bioelectric signal of the human body.

In block 620, the method 600 can utilize the alertness feature extraction model trained by self-supervised learning to obtain the feature data of the bioelectric signal used for detection of the alertness of the human body. Self-supervised learning may include the methods described above with reference to FIGS. 2-4 for training the alertness feature extraction model. In one example, the alertness feature extraction model of the method 600 may be trained with a positive sample pair generated based on a training data set. The positive sample pair may comprise a first sample randomly sampled from the training data set and a second sample with the same alertness level as the first sample. The alertness feature extraction model minimizes the distance between the first and second features extracted from the first and second samples in the alertness feature space during training. In yet another example, the alertness feature extraction model of the method 600 may be further trained with a negative sample pair generated based on a training data set. The negative sample pair includes third and fourth samples randomly sampled from the training data set, and the alertness feature extraction model maximizes the distance between the third and fourth features extracted from the third and fourth samples in the alertness feature space during training. The samples (including samples in positive sample pairs and/or negative sample pairs) generated based on the training data set for training the alertness feature extraction model may be subjected to random transform processing. Random transform processing may include one or more of shifting, trimming, scaling, and adding noise. The alertness feature extraction model may be a one-dimensional convolutional neural network (1-D CNN) as described above with reference to FIG. 5.

In block 630, the method 600 may detect the alertness of the human body using an alertness detection model based on the feature data of the bioelectric signal extracted by the alertness feature extraction model. In one example, the alertness detection model may be a classification model and the human alertness detection result may include alert (e.g., not fatigued) or not alert (e.g., fatigued). The result of the detection of the alertness of the human body may include more sub-classifications, e.g., alertness level 1, alertness level 2, alertness level 3, and so on. In another example, the alertness detection model may be a regression model and the result of the detection of the alertness of the human body may include a specific alertness value, e.g., any real value between 0 and 1.

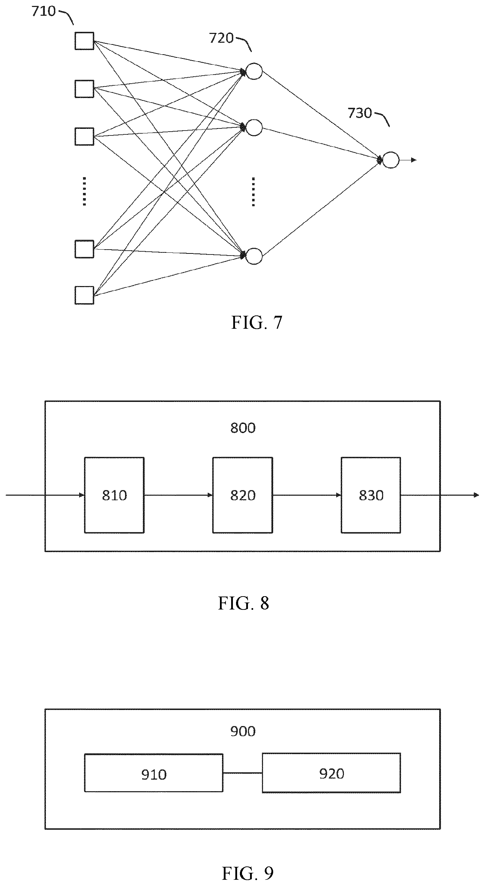

Since the alertness features of bioelectric signals extracted by the methods of the present disclosure have higher quality, the alertness detection model can adopt a simpler neural network architecture to reduce hardware costs and power consumption. FIG. 5 illustrates a schematic diagram of an alertness detection model according to one example of the present disclosure. In this example, the alertness detection model may comprise a multilayer perceptron (MLP) neural network. As shown in FIG. 7, the multilayer perceptron may comprise an input layer 710, one or more hidden layers 720, and an output layer 730. The neurons in adjacent layers of the multilayer perceptron may be fully connected.

In one example, the alertness detection model may be trained together with the alertness feature extraction model, wherein the alertness feature extraction model is pre-trained according to the self-supervised learning method described above in conjunction with FIGS. 2-4. The parameters of the alertness feature extraction model may be further optimized based on the pre-training process during training together with the alertness detection model. In another example, after training the alertness feature extraction model, the feature data extracted by the alertness feature extraction model may be utilized to perform separate training on the alertness detection model.

FIG. 8 illustrates a block diagram of a device 800 for detecting the alertness of the human body according to one example of the present disclosure. The device 800 may be a wearable device (e.g., a helmet, eyewear, watch, etc.) with the function of detecting the alertness of the human body or may be a dedicated device for detecting the alertness of the human body that can be installed on a wearable device, vehicle, etc. As shown in FIG. 8, the device 800 may comprise a receiving module 810, a feature extraction module 820, and a detection [module] 830. Further, the device 800 may further comprise a bioelectric signal collection module, an early warning module, etc.

The receiving module 810 may be used to receive a bioelectric signal for detection of the alertness of the human body. The bioelectric signal for detecting the alertness of the human body may comprise at least one of the EEG signal, EOG signal, EMG signal, etc. of the human body as described above. The receiving module 810 may receive a bioelectric signal from a bioelectric signal collection module integrated within the device 800 or may receive a bioelectric signal from outside the device 800. The feature extraction module 820 may be used to obtain the feature data of bioelectric signals for detecting the alertness of the human body using an alertness feature extraction model trained by self-supervised learning. Self-supervised learning may include the methods described above with reference to FIGS. 2-4 for training the alertness feature extraction model. The alertness feature extraction model may comprise a lightweight neural network, such as a one-dimensional convolutional neural network (1-D CNN). The detection module 830 may be used to detect the alertness of the human body based on the feature data of the bioelectric signal using the alertness detection model. The alertness detection model may comprise a lightweight neural network, such as a multilayer perceptron (MLP) neural network.

FIG. 9 illustrates a block diagram of a device 900 for detecting the alertness of the human body according to one example of the present disclosure. The device 900 may be a wearable device (e.g., a helmet, eyewear, watch, etc.) with the function of detecting the alertness of the human body or may be a dedicated device for detecting the alertness of the human body that can be installed on a wearable device, vehicle, etc. As shown in FIG. 9, the device 900 may comprise a memory 910 and at least one processor 920 coupled to the memory 910.

In one example, the device 900 may train an alertness feature extraction model for detection of the alertness of the human body and the processor 920 may be configured to perform the method described above in conjunction with FIG. 2. For example, the processor 920 may be configured to obtain a data set comprising a plurality of samples, each of which comprises a bioelectric signal of the human body and a corresponding alertness value; generate a positive sample pair based on the data set, the positive sample pair comprising a first sample randomly sampled from the data set and a second sample with the same alertness level as the first sample; train, by way of self-supervised learning, an alertness feature extraction model with the positive sample pair such that the alertness feature extraction model minimizes the distance in the alertness feature space between the first and second features extracted from the first and second samples.

In another example, the memory 910 in the device 900 may store the trained alertness feature extraction model and the alertness detection model and the processor 920 in the device 900 may be configured to execute the method described above in conjunction with FIG. 6. For example, the processor 920 may be configured to receive a bioelectric signal for detection of the alertness of the human body; use an alertness feature extraction model trained by a self-supervised learning method to obtain the feature data of the bioelectric signal for detection of the alertness of the human body; utilize the alertness detection model for detection of the alertness of the human body based on the feature data of the bioelectric signal.

The processor 920 may be a general purpose processor or may be implemented as a combination of computing devices, such as a combination of a DSP and a microprocessor, a plurality of microprocessors, a combination of one or more microprocessors and a DSP core, or any other such configuration. The memory 910 may comprise non-volatile memory for storing computer program code that implements the solution of the present disclosure and the parameters of the trained alertness feature extraction model and the alertness detection model. The memory 910 may also comprise volatile memory for temporary storage of data received during processor execution (e.g., the bioelectric signals of the human body), processed data (e.g., the feature data of bioelectric signals of the human body, the detection result of the alertness of the human body), etc.

The various operations described in conjunction with this disclosure may be performed with hardware, software executed by a processor, firmware, or any combination thereof. In one example of the present disclosure, a computer program product for detecting the alertness of the human body may comprise processor-executable computer code for executing the method 200 described above with reference to FIG. 2 and the method 600 with reference to FIG. 6. In another example of the present disclosure, a computer-readable medium may store computer program code for detecting the alertness of the human body. The computer program code, when executed by the processor, may cause the processor to perform the method 200 described above with reference to FIG. 2 and the method 600 described with reference to FIG. 6. Computer-readable media include both non-transitory computer storage media and communication media. Communication media include any medium that facilitates the transfer of a computer program from one place to another. Any connection may be appropriately referred to as a computer-readable medium. Other examples and implementations are within the scope of the present disclosure.

In addition to the content described in this document, various modifications can be made to the disclosed examples and implementations of the present invention without departing from the scope of the disclosed examples and embodiments of the present invention. Therefore, the description and examples herein should be interpreted as illustrative and not restrictive. The scope of the present invention should only be determined by reference to the claims.

## Claims

1. A method for detecting the alertness of the human body, comprising:

   obtaining a data set comprising a plurality of samples, each of which comprises a bioelectric signal of the human body and a corresponding alertness value;
   generating a positive sample pair based on the data set, the positive sample pair comprising a first sample randomly sampled from the data set and a second sample with the same alertness level as the first sample;
   training, by way of self-supervised learning, an alertness feature extraction model with the positive sample pair such that the alertness feature extraction model minimizes the distance in the alertness feature space between the first and second features extracted from the first and second samples, respectively.

2. The method according to Claim 1, wherein generating a positive sample pair based on the data set comprises:

   dividing the samples in the data set into a plurality of sample groups with different alertness levels based on the alertness values of the samples in the data set;
   randomly sampling the first sample from the plurality of sample groups;
   randomly sampling the second sample from the sample group in which the first sample is located.

3. The method according to Claim 2, wherein each sample group of the plurality of sample sets comprises an equal number of samples.

4. The method according to Claim 1, further comprising:

   generating a negative sample pair based on the data set, the negative sample pair comprising a third sample and a fourth sample randomly sampled from the data set, and,
   wherein training the alertness feature extraction model further comprises: training, by way of self-supervised learning, an alertness feature extraction model with the negative sample pair such that the alertness feature extraction model maximizes the distance in the alertness feature space between the third and fourth features extracted from the third and fourth samples, respectively.

5. The method according to Claim 4, wherein the third sample and the fourth sample have different alertness values or different alertness value levels.

6. The method according to Claim 1, further comprising:

performing random transform processing on a sample for training an alertness feature extraction model, the random transform processing comprising one or more of shifting, trimming, scaling, and adding noise; and/or generating a positive sample pair based on the data set comprises: generating a second sample by random transform processing of the first sample, the random transform processing comprising one or more of shifting, trimming, scaling, and adding noise.

7. The method according to Claim 1, wherein the alertness feature extraction model comprises a one-dimensional convolutional neural network.

8. A method for detecting the alertness of the human body, comprising:

receiving a bioelectric signal for detection of the alertness of the human body;
using an alertness feature extraction model trained by a self-supervised learning method to obtain the feature data of the bioelectric signal for detection of the alertness of the human body;
utilizing the alertness detection model for detection of the alertness of the human body based on the feature data of the bioelectric signal.

9. The method according to Claim 8, wherein the alertness feature extraction model is trained using a positive sample pair generated based on a training data set comprising a first sample and a second sample randomly sampled from the training data set, the second sample having the same alertness level as the first sample, and the alertness feature extraction model minimizes the distance in the alertness feature space between the first and second features extracted from the first and second samples, respectively, during training.

10. The method according to Claim 9, wherein the alertness feature extraction model is further trained using a negative sample pair generated based on the training data set comprising a third sample and a fourth sample randomly sampled from the training data set, and the alertness feature extraction model maximizes the distance in the alertness feature space between the third and fourth features extracted from the third and fourth samples during training.

11. The method according to any one of Claims 8 to 10, wherein the samples for training the alertness feature extraction model are subjected to random transform processing including one or more of shifting, trimming, scaling, and adding noise.

12. The method according to Claim 8, wherein the alertness feature extraction model comprises a one-dimensional convolutional neural network.

13. The method according to Claim 8, wherein the alertness detection model comprises a multilayer perceptron neural network.

14. A device for detecting the alertness of the human body, comprising:

a receiving module for receiving a bioelectric signal for detection of the alertness of the human body;
a feature extraction module for using an alertness feature extraction model trained by a self-supervised learning method to obtain the feature data of the bioelectric signal for detection of the alertness of the human body;
a detection module for utilizing the alertness detection model for detection of the alertness of the human body based on the feature data of the bioelectric signal.

15. A device for detecting the alertness of the human body, comprising:

a memory;
a processor coupled to the memory and configured to perform the method according to any one of Claims 1-13.

16. A computer program product for detecting the alertness of the human body, comprising processor-executable computer program code for performing the method according to any one of Claims 1-13.

FIG. 1

FIG. 2

FIG. 3

FIG. 4

EP 4 613 197 A1

FIG. 5

FIG. 6

EP 4 613 197 A1

FIG. 7

FIG. 8

FIG. 9

16

Europäisches
Patentamt

European
Patent Office

Office européen
des brevets

# EUROPEAN SEARCH REPORT

**Application Number**

EP 25 15 8711

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/374570 A1 (CHENG JOSEPH Y [US] ET AL) 2 December 2021 (2021-12-02) * paragraph [0026] - paragraph [0028] * * paragraph [0036] * * paragraph [0052] - paragraph [0054] * * paragraph [0077] * | 1-16 | INV. A61B5/16 A61B5/372 A61B5/397 A61B5/398 A61B5/00 G06N20/00 |
| X | US 2023/306267 A1 (JACOB BANVILLE HUBERT [CA] ET AL) 28 September 2023 (2023-09-28) * paragraph [0003] * * paragraph [0136] - paragraph [0137] * * paragraph [0194] - paragraph [0198] * * paragraph [0277] * | 1,4-12, 14-16 | |
| A | | 2,3,13 | |
| A | US 6 070 098 A (MOORE-EDE MARTIN C [US] ET AL) 30 May 2000 (2000-05-30) * column 10, line 9 - line 47 * | 1-16 | |
| A | YUNFEI GUO ET AL: "Emotion recognition based on multi-modal electrophysiology multi-head attention Contrastive Learning", ARXIV.ORG, CORNELL UNIVERSITY LIBRARY, 201 OLIN LIBRARY CORNELL UNIVERSITY ITHACA, NY 14853, 12 July 2023 (2023-07-12), XP091578561, * Chapter III - Methods * | 1-16 | |

**TECHNICAL FIELDS SEARCHED (IPC)**

A61B
G06N

The present search report has been drawn up for all claims

| Place of search | Date of completion of the search | Examiner |
|---|---|---|
| The Hague | 14 April 2025 | Bourquin, Yannyk |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 25 15 8711

This annex lists the patent family members relating to the patent documents cited in the above-mentioned European search report.
The members are as contained in the European Patent Office EDP file on
The European Patent Office is in no way liable for these particulars which are merely given for the purpose of information.

14-04-2025

| Patent document cited in search report | Publication date | Patent family member(s) | Publication date |
|---|---|---|---|
| US 2021374570 A1 | 02-12-2021 | NONE | |
| US 2023306267 A1 | 28-09-2023 | CA 3189784 A1 | 03-02-2022 |
| | | EP 4189607 A1 | 07-06-2023 |
| | | US 2023306267 A1 | 28-09-2023 |
| | | WO 2022020968 A1 | 03-02-2022 |
| US 6070098 A | 30-05-2000 | US 6070098 A | 30-05-2000 |
| | | US 6511424 B1 | 28-01-2003 |

EPO FORM P0459

For more details about this annex : see Official Journal of the European Patent Office, No. 12/82